# EUROPÄISCHE PATENTSCHRIFT

(11) **EP 2 341 340 B1**
(45) Veröffentlichungstag und Bekanntmachung des Hinweises auf die Patenterteilung: **19.08.2015**
(21) Anmeldenummer: 10016230.4
(22) Anmeldetag: 31.12.2010
(51) Int. Cl.: G01N 33/28

(54) **Vorrichtung zur Messung von Ölzustandsänderungen**
Device for measuring oil state changes
Dispositif de mesure de variations de l'état d'huile

(30) Priorität: 31.12.2009 DE 202009017711 U
(43) Veröffentlichungstag der Anmeldung: 06.07.2011
(73) Patentinhaber: Vogt, Joachim, 89079 Ulm-Unterweiler (DE); Kalunka, Daniel, 73760 Ostfildern (DE)
(72) Erfinder: Vogt, Joachim, 89079 Ulm-Unterweiler (DE); Kalunka, Daniel, 73760 Ostfildern (DE)
(74) Vertreter: Fiener, Josef

(56) Entgegenhaltungen:
- EP-A1- 0 635 714
- EP-A2- 0 330 522
- EP-A2- 2 096 434
- WO-A1-96/28742
- DE-A1-102007 042 109
- US-A1- 2003 005 751
- US-A1- 2003 145 647

## Beschreibung

Die Erfindung betrifft eine Messvorrichtung zur Fluidzustandsänderung mit den oberbegrifflichen Merkmalen des Anspruchs 1, vorzugsweise von Motoröl in einem Kraftfahrzeug.

Eine derartige Messvorrichtung ist aus der DE 10 2007 042 109 A1 (und in ähnlicher Form aus der US 2003/005 751 oder EP 2 096 434 A2) bekannt. In Geräten und Antriebseinheiten (z. B. Motoren, Getrieben usw.) insbesondere mit beweglichen Teilen, die mit verschiedenen Betriebs- und Schmiermitteln befüllt sind, ist eine Überwachung der Fluidqualität, insbesondere der sog. Alterung des Betriebsmittels wichtig für die Lebensdauer. Die Fluidqualität muss sich innerhalb festgelegter Grenzen bewegen, um eine verschleißarme und somit wartungsarme Funktion der Geräte bzw. Antriebe zu gewährleisten. Daneben muss sichergestellt werden, dass für die Fluidmenge tolerierbare Minimal- und Maximalgrenzwerte weder unter- noch überschritten werden. Der eingangs genannte Stand der Technik gibt hierzu keine weiteren Informationen, auch nicht weiterhin bekannte Messsysteme zur Erfassung der Ölqualitäten, wie bei der WO 96, 28742 A1, US 2003/145647 A1, EP 0 330 522 A1 oder EP 0635 714.

Oft kann die Überwachung der Fluidqualität nur in Laboren mit aufwändigen Sensoren durchgeführt werden. Diese sind allerdings teuer oder überwachen teilweise nur einzelne physikalische Eigenschaften des Öls (z. B. die Viskosität) und teilweise nur zu einem bestimmten Zeitpunkt, nämlich erst nach Entnahme aus dem Motor, Getriebe etc., obwohl eine Überwachung direkt in der Maschine wünschenswert wäre.

Da sich Getriebe- oder Motoröle (auch schon Frischöle) physikalisch und chemisch über den Alterungsprozess bei dem Einsatz in einem Motor unterschiedlich verhalten (auch abhängig von den Additiven im Öl), sollte oftmals nicht nur ein einzelner Messwert oder nur zu einem bestimmten Zeitpunkt gemessen werden. Daher ist auch die Veränderung aller Messwerte über die Zeit von Bedeutung und auch der direkte Vergleich mit den Öl-Ausgangsmessgrößen (z.B. nach einem Ölwechsel).

Daher liegt der Erfindung die Aufgabe zugrunde, eine Messvorrichtung zur Fluidzustandsänderung, insbesondere zur Überwachung der Ölqualität in einem Fahrzeug auf einfache Weise zu schaffen.

Diese Aufgabe wird gelöst durch eine Messvorrichtung mit den Merkmalen des Anspruchs 1. Vorteilhafte Weiterbildungen der Erfindung sind Gegenstand der abhängigen Ansprüche.

Die erfindungsgemäße Messvorrichtung eignet sich zur Überwachung von Fluiden und hierbei insbesondere für Betriebs- und Schmiermitteln in einem Antrieb oder einem Gerät, vorzugsweise in einem Fahrzeugmotor. Nachfolgend bezieht sich die Beschreibung nur noch auf die Betriebsmittel bzw. Motoröl, wobei diese dann Schmiermittel sowie alle übrigen Fluide in einem Antrieb oder in einem Gerät umfassen, deren Qualität für den reibungslosen und wartungsarmen Betrieb eines Antriebs oder Gerätes von Bedeutung sind.

Mit der erfindungsgemäßen Messvorrichtung wird die Möglichkeit geschaffen, auf eine einfache Art und Weise die für die Ölqualität schädliche Faktoren, schlechte Fliessfähigkeit des Motoröls oder auch schlecht verbrannter Kraftstoff zu überwachen. Dies erfolgt durch einfache Nutzung einer ohnehin vorhandenen Fluid- oder Ölpumpe, deren Leistungsaufnahme als Maß für die Ölqualität überwacht wird. Dies gestaltet sich besonders einfach für eine elektrische Ölpumpe, deren Stromaufnahme gemessen wird und damit aussagekräftige Messwerte für die Ölqualität (Alterung, Viskosität etc.) erhalten werden. Der Füllgrad des Ölpumpenansaugrohrs kann mit entsprechenden Sensoren gemessen werden, insbesondere dessen Schwingungsintensität. Füllt sich das Ansaugrohr schlecht oder gar nicht, ist bei entsprechender Temperatur zähes oder gar kein Öl vorhanden. Ist das Ansaugrohr andererseits halbleer, weist dieses beim Ölansaugen ein anderes Schwingungsverhalten als bei einem vollen Ansaugrohr.

Zunächst sind dazu alle relevanten Messgrößen z. B. bei einem Ölwechsel zurückzusetzen, so dass sich das System auf das neu eingefüllte Motoröl einstellen kann. Die immer wieder gemessenen Werte werden dann kontinuierlich oder diskontinuierlich verglichen. Werden Extremwerte oder Abweichungen festgestellt, kann eine Warnung z. B. für den Fahrer ausgelöst werden oder die verbleibende Reststrecke/-zeit bis zum nächsten Ölwechsel (z. B. per Anzeige auf einem Display) wird dementsprechend reduziert.

Neben der Überwachung der Leistungsaufnahme der Fluid- bzw. Ölpumpe und des Füllgrads und/oder Luftgehalts des Fluids im Ansaugrohr des Fluidpumpe können folgende Messwerte zusätzlich erfasst werden:
- Über einen Füllstandssensor kann der sich durch Kraftstoffeintrag (z. B. über die Kolbenringe) langsam erhöhende Füllstand über eine bestimmte Zeit oder Wegstrecke gemessen werden Hierzu kann auch ein schon im Fahrzeug befindlicher Sensor verwendet werden. Die Sicherstellung der richtigen Motorlage für die Messung erfolgt z. B. über Winkel- und Querbeschleunigungssensoren. Erhöht sich nun die Ölvolumenmenge im Fahrzeug (bzw. deren Ölwanne) über die Zeit, kann von einem Kraftstoffeintrag ausgegangen werden und damit von einem kürzeren Wartungsintervall aufgrund der Ölverdünnung.
- Messwerte, die aus dem direkten oder indirekten Kontakt mit dem Verbrennungsvorgang im Motor entstehen, beispielsweise sog. Tank- oder Kraftstoffsensoren, die "schlechten" Kraftstoff detektieren. Wird Kraftstoff mit bestimmten Zusätzen oder Additiven getankt, so kann hier ein entsprechender Abzug der erlaubten Laufstrecke bis zum nächsten Ölwechsel erfolgen (z. B. ein bestimmter Laufstreckenabzug pro Liter schlechtem Kraftstoff).
- Über Ölzusatzfilter oder z. B. Gitter in Ölleitungen, die bei sehr schlechtem bzw. stark verschmutztem ÖI nach einer gewissen Zeit "zumachen" und somit im Nebenschluss eine schlechte Ölqualität anzeigen. Auch die Anhaftung an bestimmten Materialien kann hierzu als Indikator verwendet werden, da sich nur gealtertes Öl chemisch auf bestimmten Materialien absetzt. Dadurch ändert sich der Öldurchfluss in den entsprechenden Ölleitungen und kann durch Drucküberwachung detektiert werden.
- Die zeitliche Füllstandsänderung bei Motorstart, da bei zähem Öl nur ein langsames Abpumpen aus der Ölwanne erfolgt (Füllstand ändert sich extrem langsam).
- Der Kurbelgehäusedruck im Motor, da bei einem starken Anstieg nicht mehr von einer ordnungsgemäßen Trennung des Verbrennungsraums mit dem Kurbelgehäuse ausgegangen werden (z. B. beschädigte Kolbenringe, die zu starken Kraftstoffeinträgen ins Motoröl führen).
- Die Messwerte an einem Wärmetauscher und/oder im Ölkreislauf werden direkt oder indirekt aufgenommen werden und/oder in Relation zu anderen vorhandenen Messgrößen gesetzt wird (u.a. Motorlast, Kühlmitteltemperatur, Motorleistung, Kraftstoffverbrauch usw.).

Diese zusätzlichen Messwerte werden bevorzugt in einem Steuergerät gespeichert und immer wieder verglichen. Eine Warnung o.ä. kann über schon vorhandene Anzeigen im Fahrzeug oder an der Maschine erfolgen.

Nachfolgend wird anhand der Zeichnungen die Erfindung näher erläutert. Hierbei zeigen:
Fig. 1 eine Schemadarstellung eines Motors mit Ölpumpe,
Fig. 2 eine schematische Ansicht eines Tanks mit Sensor,
Fig. 3 eine Schemadarstellung eines Ölkreislaufs,
Fig. 4 eine weitere Skizze eines Ölkreislaufs zu einem Ventiltrieb des Motors,
Fig. 5 eine Schemadarstellung ähnlich zu Fig. 3, und
Fig. 6 eine schematische Ansicht der Messvorrichtung mit Auswerte-/Anzeigegeräten.

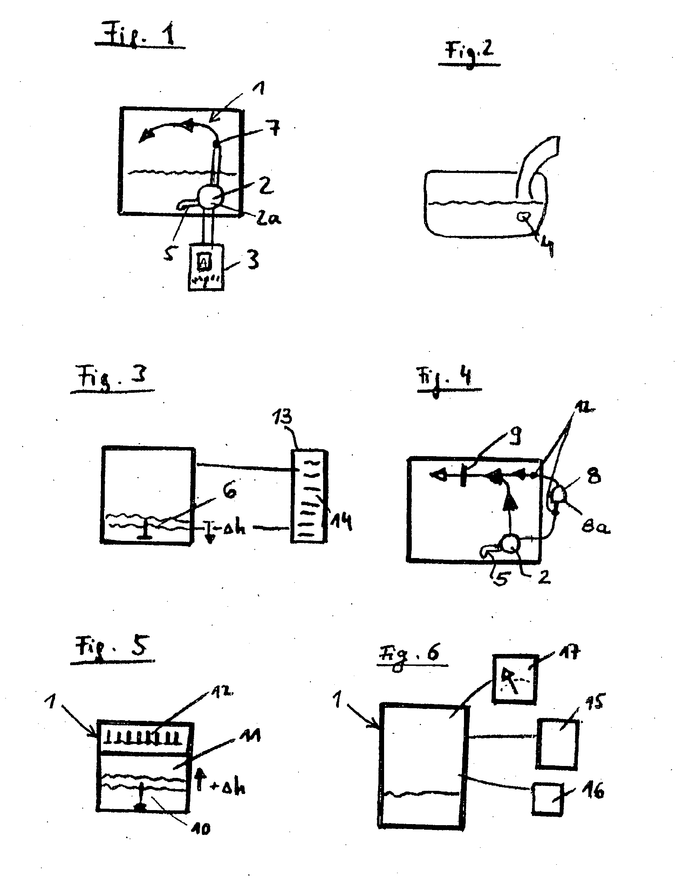

In Fig. 1 ist eine Messvorrichtung zur Fluidzustandsänderung am Beispiel eines Motors dargestellt. Hierbei erfolgt für Betriebs - und/ oder Schmiermittel in einem Antrieb oder Gerät 1 vorzugsweise die Zustandskontrolle von Motoröl durch die Überwachung/Messung der Leistungsaufnahme einer Fluidpumpe 2, insbesondere der Stromaufnahme 3 (hier angedeutet durch ein Amperemeter) einer elektrisch angetriebenen Ölpumpe. Solche elektrischen Ölpumpen werden vermehrt eingesetzt und erlauben in besonders einfacher Weise die Messung der Leistungsaufnahme als direktes Maß für die Ölqualität. Auch bei bei einer mechanisch angetriebenen Ölpumpe kann dies auf relativ einfache Weise mit einem Drehmoment-Sensor 2a erfolgen, z. B. mit der Dehnmessstreifentechnik auf der Antriebswelle der Ölpumpe 2.

In Fig. 2 ist ein Fahrzeugtank angedeutet, in dem ein Kraftstoffsensor 4 eingesetzt ist. Gemessen werden kann auch in oder an allen kraftstoffführenden Bauteilen, z. B. dem Tankstutzen oder an Kraftstoffpumpen-Leitungen. Die dadurch erfassten Messwerte zur Qualität des Kraftstoffes (insbesondere qualitätssenkende Zusätze) können somit in die Berechnung eines Ölwechselintervalls miteinbezogen werden. In Fig. 1 ist auch die Möglichkeit angedeutet, dass der Füllgrad und/oder Luftgehalt des Öls im Ansaugrohr 5 der Ölpumpe 2 überwacht wird, insbesondere durch Messung des Schwingungsverhaltens des Ansaugrohrs 5, um weitere Indikatorwerte für die Ölqualität abzuleiten.

In Fig. 3 ist eine Messvorrichtung dargestellt, die zusätzlich die Füllstandsänderung Δh durch einen Füllstandssensor 6, insbesondere beim Motorstart misst. Zudem kann mindestens ein zusätzlicher Sensor (7) für die Öltemperatur vorhanden sein, wie in Fig. 1 eingezeichnet.

In Fig. 4 ist die Messvorrichtung mit einem Zusatzbehälter 8 gekoppelt, in dem Zustandsänderungen des Fluids bzw. Öls mit Sensoren 8a, insbesondere für Druck und/oder Temperaturverlauf gemessen werden. Im oberen Bereich, z. B. im Zylinderkopf oder am Ventiltrieb 12 werden im Ölkreislauf Materialien 9, insbesondere in Siebform verwendet werden, an denen gealterte Ölbestandteile durch chemische Reaktion anhaften können und somit ein Indiz für die nachlassende Ölqualität liefern.

In Fig. 5 wird eine Zunahme des Ölfüllstands (wiederum mit Δh bezeichnet) mit einem Füllstandssensor 10 während des Motorbetriebs gemessen wird. Zudem kann auch der Kurbelgehäusedruck mit einem entsprechenden Drucksensor 11 gemessen werden. Weiterhin können Ventilöffnungszeiten und/oder Hubwege eines Ventiltriebs 12 berücksichtigt werden. Dies ist in Fig. 5 im oberen Bereich für eine obenliegende Nockenwelle angedeutet. Natürlich kann der Ventiltrieb 12 bzw. die Nockenwelle auch untenliegend sein, wie dies in Fig. 4 durch die tiefere Bezugslinie angedeutet ist. Der Durchsatz und/oder die Durchflussgeschwindigkeit kann auch an einem Ölzusatzfilter, insbesondere im Bereich des Ventiltriebs 12 gemessen werden.

Wie in Fig. 3 schematisch dargestellt, können die Messwerte auch an einem Wärmetauscher 13 und/oder im Ölkreislauf 14, insbesondere an einem Ölkühler aufgenommen werden und in Relation zu anderen Motor-Messgrößen gesetzt werden, insbesondere zur Motorlast, Kühlmitteltemperatur, Motorleistung oder zum Kraftstoffverbrauch.

In Fig. 6 ist schließlich die Ankoppelung der Messvorrichtung für das Motoröl zu wenigstens einem Steuergerät 15 oder einer weiteren Erfassungs- und Auswerteeinheit 16 gezeigt. Zudem ist bevorzugt wenigstens eine Anzeige 17 vorgesehen ist, die dem Steuergerät 15 und/oder der Auswerteeinheit 16 direkt oder indirekt gekoppelt ist, um dem Fahrer (oder Maschinenbediener) Hinweise auf die Ölqualität und zu erwartende Ölwechselintervalle zu geben.

## Patentansprüche

1. Messvorrichtung zur Fluidzustandsänderung, insbesondere für Betriebs - und/ oder Schmiermittel in einem Antrieb oder in einem Gerät (1), vorzugsweise von Motoröl, Überwachung eingerichtet zur Messung der Leistungsaufnahme einer Fluidpumpe (2), insbesondere der Stromaufnahme (3) einer elektrisch angetriebenen Ölpumpe,
**dadurch gekennzeichnet, dass** die Messvorrichtung dazu eingerichtet ist, den Füllgrad und/oder Luftgehalt des Fluids im Ansaugrohr (5) der Fluidpumpe (2) zu überwachen.

2. Messvorrichtung nach Anspruch 1, wobei bei einer mechanisch angetriebenen Ölpumpe ein Drehmoment-Sensor (2a) vorgesehen ist.

3. Messvorrichtung nach Anspruch 1 oder 2, so eingerichtet, dass Messwerte eines Kraftstoffsensors (4) im Tank oder in kraftstoffführenden Teilen in die Berechnung eines Ölwechselintervalls miteinbezogen sind.

4. Messvorrichtung nach einem der Ansprüche 1 bis 3, so eingerichtet, dass der Füllgrad und/oder Luftgehalt des Fluids im Ansaugrohr (5) der Fluidpumpe (2) durch Messung des Schwingungsverhaltens des Ansaugrohrs (5). überwacht wird.

5. Messvorrichtung nach einem der Ansprüche 1 bis 4, so eingerichtet, dass die Füllstandsänderung (6) beim Motorstart gemessen wird.

6. Messvorrichtung nach einem der Ansprüche 1 bis 5, wobei mindestens ein zusätzlicher Sensor (7) für die Öltemperatur vorhanden ist.

7. Messvorrichtung nach einem der Ansprüche 1 bis 6, wobei ein Zusatzbehälter (8) vorhanden ist, in dem Fluidzustandsänderungen mit Sensoren (8a), insbesondere für Druck und/oder Temperaturverlauf gemessen werden können.

8. Messvorrichtung nach einem der Ansprüche 1 bis 7, so eingerichtet, dass eine Zunahme des Ölfüllstands mit einem Füllstandssensor (10) während des Motorbetriebs gemessen wird.

9. Messvorrichtung nach einem der Ansprüche 1 bis 8, so eingerichtet, dass der Kurbelgehäusedruck (11) gemessen wird.

10. Messvorrichtung nach einem der Ansprüche 1 bis 9, so eingerichtet, dass der Durchsatz und/ oder die Durchflussgeschwindigkeit durch einen Ölzusatzfilter, insbesondere im Bereich des Ventiltriebs (12) gemessen wird.

11. Messvorrichtung nach einem der Ansprüche 1 bis 10, wobei die Messvorrichtung für die Erfassung von Messwerten mit wenigstens einem Steuergerät (15) oder einer weiteren Erfassungs- und Auswerteeinheit (16) gekoppelt ist.

12. Messvorrichtung nach Anspruch 11, wobei wenigstens eine Anzeige (17) vorgesehen ist, die dem Steuergerät (15) und/oder der Auswerteeinheit (16) direkt oder indirekt gekoppelt ist.

## Claims

1. Measuring device for fluid condition change, in particular for operation fluids and/or lubricants in a drive or a device (1), preferably of engine oil, adapted for monitoring/ measuring the power consumption of a fluid pump (2), in particular the current consumption (3) an electrically driven oil pump.
**characterized in that**
the measuring device is adapted to monitor the filling degree and/or air content of the fluid in the intake pipe (5) of the fluid pump (2).

2. Measuring device according to claim 1, wherein a torque sensor (2a) is provided for a mechanically driven oil pump.

3. Measuring device according to claim 1 or 2, adapted to incorporate measured values of a fuel sensor (4) in the tank or fuel-transporting parts in the calculation of an oil change interval.

4. Measuring device according to one of claims 1 to 3, adapted to measure the filling degree and/or air content of the fluid in the intake pipe (5) of the fluid pump (2) via the oscillation behaviour of the intake pipe (5).

5. Measuring device according to one of claims 1 to 4, adapted to measure the level change (6) when the engine starts.

6. Measuring device according to one of claims 1 to 5, wherein at least one additional sensor (7) is provided for the oil temperature.

7. Measuring device according to one of claims 1 to 6, wherein an additional container (8) is provided, in which fluid state change can be measured by sensors (8a), in particular for pressure and/or temperature profile.

8. Measuring device according to one of claims 1 to 7, adapted to measure an increase in the oil level with a level sensor (10) during engine operation.

9. Measuring device according to one of claims 1 to 8, adapted to measure the crankcase pressure (11).

10. Measuring device according to one of claims 1 to 9, adapted to measure the throughput and/or the flow rate of an oil additive filter, in particular in the area of the valve drive (12).

11. Measuring device according to one of claims 1 to 10, wherein the measuring device for the acquisition of measured values is coupled with at least one control unit (15) or a further detection and evaluation unit (16).

12. Measuring device according to claim 11, wherein at least one display (17) is provided, which is coupled to the control device (15) and/or the evaluation unit (16), directly or indirectly.

## Revendications

1. Dispositif de mesure de la variation de l'état d'un fluide, en particulier pour des produits consommables et/ou lubrifiants dans un entraînement ou dans un appareil (1), de préférence d'huile moteur, conçu pour surveiller/mesurer la consommation de puissance d'une pompe à fluide (2), en particulier la consommation de courant (3) d'une pompe à huile à entraînement électrique,
**caractérisé en ce que** le dispositif de mesure est conçu pour surveiller le degré de remplissage et/ou la teneur en air du fluide dans le tube d'aspiration (5) de la pompe à fluide (2).

2. Dispositif de mesure selon la revendication 1, dans lequel, dans le cas d'une pompe à huile entraînée mécaniquement, un capteur de couple (2a) est prévu.

3. Dispositif de mesure selon la revendication 1 ou 2, lequel est conçu de façon que des valeurs de mesure d'un capteur de carburant (4) présent dans le réservoir ou dans des parties conduisant le carburant soient également prises en compte dans le calcul d'un intervalle de vidange d'huile.

4. Dispositif de mesure selon l'une des revendications 1 à 3, lequel est conçu de façon que le degré de remplissage et/ou la teneur en air du fluide dans le tube d'aspiration (5) de la pompe à fluide (2) soient surveillés par mesure du comportement vibratoire du tube d'aspiration (5).

5. Dispositif de mesure selon l'une des revendications 1 à 4, lequel est conçu de façon que la variation du niveau de remplissage (6) soit mesurée au démarrage du moteur.

6. Dispositif de mesure selon l'une des revendications 1 à 5, dans lequel au moins un capteur supplémentaire (7) pour la température d'huile est présent.

7. Dispositif de mesure selon l'une des revendications 1 à 6, dans lequel un réservoir supplémentaire (8) est présent, dans lequel des variations de l'état du fluide peuvent être mesurées avec des capteurs (8a), en particulier de pression et/ou de variation de température.

8. Dispositif de mesure selon l'une des revendications 1 à 7, lequel est conçu de façon qu'une augmentation du niveau de remplissage d'huile soit mesurée avec un capteur de niveau de remplissage (10) pendant le fonctionnement du moteur.

9. Dispositif de mesure selon l'une des revendications 1 à 8, lequel est conçu de façon que la pression du carter de moteur (11) soit mesurée.

10. Dispositif de mesure selon l'une des revendications 1 à 9, lequel est conçu de façon que le débit et/ou la vitesse d'écoulement à travers un filtre à huile supplémentaire, en particulier dans la zone de la commande de soupapes (12), soient mesurés.

11. Dispositif de mesure selon l'une des revendications 1 à 10, lequel est couplé, pour l'acquisition de valeurs de mesure, à au moins un appareil de commande (15) ou une autre unité d'acquisition et d'évaluation (16).

12. Dispositif de mesure selon la revendication 11, dans lequel au moins un indicateur (17) est prévu, qui est couplé directement ou indirectement à l'appareil de commande (15) et/ou à l'unité d'évaluation (16).
